(19) Europäisches Patentamt
European Patent Office
Office européen des brevets

(11) **EP 2 662 068 B1**

(12) **EUROPEAN PATENT SPECIFICATION**

(45) Date of publication and mention of the grant of the patent:
**05.07.2017 Bulletin 2017/27**

(21) Application number: **11854607.6**

(22) Date of filing: **24.11.2011**

(51) Int Cl.:
***A61K 8/06*** (2006.01) ***A61K 8/37*** (2006.01)
***A61K 8/44*** (2006.01) ***A61K 8/60*** (2006.01)
***A61K 9/107*** (2006.01) ***A61K 47/26*** (2006.01)
***A61Q 19/00*** (2006.01)

(86) International application number:
**PCT/JP2011/076994**

(87) International publication number:
**WO 2012/093524 (12.07.2012 Gazette 2012/28)**

(54) **OIL-IN-WATER EMULSION COMPOSITION**

ÖL-IN-WASSER-EMULSIONSZUSAMMENSETZUNG

COMPOSITION D'ÉMULSION D'HUILE DANS DE L'EAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.01.2011 JP 2011001094**

(43) Date of publication of application:
**13.11.2013 Bulletin 2013/46**

(73) Proprietor: **Kao Corporation**
**Chuo-Ku**
**Tokyo 103-8210 (JP)**

(72) Inventor: **HAYASE, Motoi**
**Odawara-shi**
**Kanagawa 250-0002 (JP)**

(74) Representative: **Hoffmann Eitle**
**Patent- und Rechtsanwälte PartmbB**
**Arabellastraße 30**
**81925 München (DE)**

(56) References cited:
**WO-A2-2007/122822** **JP-A- 2009 149 566**
**JP-A- 2009 149 567** **JP-A- 2009 167 158**
**JP-A- 2009 167 159** **JP-A- 2009 275 017**
**JP-A- 2011 168 548**

- **TOKUMA FUKUOKA: 'Expansion of structures and functions of novel glycolipid biosurfactants' OLEOSCIENCE vol. 9, no. 4, 01 April 2009, pages 127 - 133, XP055118251**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 662 068 B1

## Description

[Field of the Invention]

The present invention relates to an oil-in-water emulsion composition which is excellent in a touch feeling.

[Background of the Invention]

Oil-in-water emulsion compositions are superior to water-in-oil emulsion compositions in feel upon application to the skin, and thus have been used as sunscreens in recent years. However, UV absorbers used in sunscreens have a poor solubility in solvents and are insoluble in moisturizing agents such as glycerol or water, and therefore have been difficult to blend stably in oil-in-water emulsion compositions. Accordingly, oil-in-water emulsion formulations have been studied using surfactants having an alkylene oxide group which have a relatively good compatibility with UV absorbers (see Patent Literature 1).

Surfactants having an alkylene oxide group such as an ethylene oxide, propylene oxide or butylene oxide group in the molecule have hitherto been widely used in applications including cosmetics and pharmaceuticals. However, their use has been called into question in recent years in terms of safety and environmental burdens. Formulations using surfactants free of alkylene oxide groups such as polyglycerol fatty acid ester and sucrose fatty acid ester are being studied in the development of cosmetics (see Patent Literatures 2 to 4).

However, use of such highly hydrophilic surfactants such as polyglycerol fatty acid ester and sucrose fatty acid ester causes not only functional problems such as low water resistance but also stickiness or the like in the coatings, resulting in both decreased water resistance and decreased feeling, disadvantageously. This tendency is particularly significant in cases such as cosmetics and pharmaceuticals where such surfactants are present together with other water-soluble active ingredients. Therefore, for formulations blended with a large amount of an UV absorber, it has been difficult to prepare oil-in-water emulsion compositions superior in a touch feeling and superior in water resistance and stability without using surfactants having an alkylene oxide group.

In recent years, mannosyl erythritol lipids have been developed as one kind of novel biosurfactants, and are known as skin-friendly surfactants (see Patent Literatures 5 to 7).

[Citation List]

[Patent Literature]

[Patent Literature 1] JP-A-2009-102236
[Patent Literature 2] JP-A-2007-153858
[Patent Literature 3] JP-A-11-262653
[Patent Literature 4] JP-A-11-71261
[Patent Literature 5] JP-A-2009-167159
[Patent Literature 6] JP-A-2009-149566
[Patent Literature 7] JP-A-2009-149567

[Summary of the Invention]

[Technical Problem]

The present invention provides an oil-in-water emulsion composition, comprising the following components (A) to (C):

(A) an UV absorber of the following (i) and/or (ii):

(i) at least one methoxycinnamic acid derivatives selected from the group consisting of ethyl p-methoxycinnamate, isopropyl p-methoxycinnamate, 2-ethoxyethyl p-methoxycinnamate, 2-ethylhexyl p-methoxycinnamate, sodium p-methoxycinnamate, potassium p-methoxycinnamate and glyceryl mono-2-ethylhexanoate di-p-methoxycinnamate,
(ii) at least one benzoic acid derivatives selected from the group consisting of p-aminobenzoic acid, ethyl p-aminobenzoate, glyceryl p-aminobenzoate, octyl p-aminobenzoate, amyl p-dimethylaminobenzoate, 2-ethylhexyl p-dimethylaminobenzoate and ethyl 4-[N,N-di(2-hydroxypropyl)amino]benzoate,

(B) a sorbitan fatty acid ester having an HLB of 6 or less, and
(C) a mannosyl erythritol lipid,

wherein the surfactants in the composition have a combined HLB of less than 8, and a content of the component (A) is 75 parts by mass or more relative to 100 parts by mass of a total amount of oily components.

[Effects of the Invention]

The oil-in-water emulsion composition of the present invention may be used for purposes such as cosmetics and pharmaceuticals superior in a feel upon application (spreadability, freshness), and can be suitably applied, in particular, as skin external preparations for sun protection such as sunscreen, makeup base, foundation and lip cream.

[Embodiments for carrying out the Invention]

The problem is that use of the mannosyl erythritol lipid alone is difficult to form oil-in-water emulsion compositions.
Therefore, according to the present invention, there is provided a stable oil-in-water emulsion composition blended with an UV absorber which is excellent in a feel upon application, using a mannosyl erythritol lipid.
The present inventor has studied on emulsion systems by combining a mannosyl erythritol lipid with various emulsifiers and UV absorbers in this context, and as a result, quite unexpectedly found that, in contrast to the fact that water-in-oil emulsion compositions are typically formed in case of using low-HLB surfactants, a stable oil-in-water emulsion composition can be provided by using a mannosyl erythritol lipid in combination with a sorbitan fatty acid ester having an HLB of 6 or less and an UV absorber of methoxycinnamic acid derivatives and/or benzoic acid derivatives and setting the combined HLB of the surfactants in the composition to be less than 8, and thus an oil-in-water emulsion composition excellent in feel upon application and superior in stability can be provided. This finding has led to the completion of the present invention.
As (A) an UV absorber of the present invention, (i) methoxycinnamic acid derivatives and/or (ii) dimethylbenzoic acid derivatives are used. (i) Examples of methoxycinnamic acid derivatives can include ethyl p-methoxycinnamate, isopropyl p-methoxycinnamate, 2-ethoxyethyl p-methoxycinnamate, 2-ethylhexyl p-methoxycinnamate, sodium p-methoxycinnamate, potassium p-methoxycinnamate and glyceryl mono-2-ethylhexanoate di-p-methoxycinnamate. (ii) Examples of dimethylbenzoic acid derivatives can include p-aminobenzoic acid, ethyl p-aminobenzoate, glyceryl p-aminobenzoate, octyl p-aminobenzoate, amyl p-dimethylaminobenzoate, 2-ethylhexyl p-dimethylaminobenzoate and ethyl 4-[N,N-di(2-hydroxypropyl)amino]benzoate. One or more of these can be used in combination. Of these UV absorbers, preferred examples can include 2-ethylhexyl p-methoxycinnamate and 2-ethylhexyl p-dimethylaminobenzoate which have a good solubility in aqueous components, and 2-ethylhexyl p-methoxycinnamate is more preferable due to its little odor.
These methoxycinnamic acid derivatives are well-known substances and for example, Uvinul MC 80 (manufactured by BASF) and Parsol MCX (manufactured by DSM Nutritional Products) can be used as 2-ethylhexyl p-methoxycinnamate. Nomcort X (manufactured by The Nisshin Oillio Group, Ltd.) can be used as glyceryl mono-2-ethylhexanoate di-p-methoxycinnamate, and Escalol 507 (manufactured by ISP Japan Ltd.) can be used as 2-ethylhexyl p-dimethylaminobenzoate, and so on.
The content of the component (A) in the oil-in-water emulsion composition of the present invention is preferably 1 to 40 mass%, more preferably 2 to 35 mass%, even more preferably 3 to 25 mass%, to provide a stable oil-in-water emulsion system and in terms of oiliness.
The content of the component (A) in the oil-in-water emulsion composition of the present invention is preferably 75 parts by mass or more, more preferably 80 to 100 parts by mass, even more preferably 85 to 100 parts by mass relative to 100 parts by mass of the total amount of the oily components, to provide a stable oil-in-water emulsion system and achieve an excellent feel upon application.
Oily components other than the UV absorber which may be comprised in the oily components of the oil-in-water emulsion cosmetic composition of the present invention include squalanes such as olive squalane, rice squalane and shark squalane; silicone oils such as dimethylpolysiloxane and cyclic silicone; hydrocarbons such as paraffin, liquid paraffin, vaseline, olefin oligomers and squalane; vegetable oils such as jojoba oil, olive oil and macadamia nut oil; triglycerides such as glyceryl tri-2-ethylhexanoate and glyceryl triisostearate; waxes such as yellow beeswax, Japan wax and carnauba wax; ester oils such as octyldodecyl myristate, cetyl palmitate, isostearyl isostearate and isopropyl myristate; higher alcohols such as cetanol, behenyl alcohol, isostearyl alcohol, jojoba alcohol, oleyl alcohol, stearyl alcohol and branched long-chain aliphatic alcohol; sterols and derivatives thereof such as cholesterol, phytosterol, branched fatty acid cholesterol ester and macadamia nut fatty acid phytosteryl ester; processed oils such as hydrogenated

oils; higher fatty acids such as stearic acid, myristic acid, long-chain iso fatty acid and long-chain anteiso fatty acid; ethers such as dicapryl ether; and terpenes such as limonene and hydrogenated bisabolol.

The content of the oily component (including the component (A)) in the oil-in-water emulsion cosmetic composition of the present invention is preferably 1 to 40 mass%, more preferably 2 to 35 mass%, even more preferably 3 to 30 mass%, in terms of smooth feel upon application and oiliness.

In the present invention, a stable oil-in-water emulsion composition can be obtained using (B) a sorbitan fatty acid ester having an HLB of 6 or less in addition to (C) a mannosyl erythritol lipid which is one kind of a biosurfactant.

The HLB value is an index indicating the ratio of relative affinities of a surfactant for both liquids in an oil-water system, generally, surfactants having a low HLB value (particularly an HLB of about 3 to 6) are highly lipophilic and tend to produce water-in-oil emulsion compositions. Surfactants having a high HLB value (particularly an HLB of about 8 to 18) are highly hydrophilic and tend to produce oil-in-water emulsion compositions. Sorbitan fatty acid esters having an HLB of 6 or less which are used in the oil-in-water emulsion compositions of the present invention are generally used as emulsifiers for water-in-oil emulsion composition due to their low HLB values. However, quite unexpectedly, stable oil-in-water emulsion compositions can be obtained using them in combination with (C) a mannosyl erythritol lipid.

Here, the "HLB" (Hydrophilic-Lipophilic Balance) represents the molecular weight of the hydrophilic group moiety relative to the total molecular weight of the surfactant, and the HLB for nonionic surfactants can be determined according to Griffin’s formula.

Examples of the sorbitan fatty acid ester having an HLB of 6 or less used in the present invention include sorbitan monostearate (HLB = 4.7), sorbitan monoisostearate (HLB = 5), sorbitan sesquistearate (HLB = 4.2), sorbitan sesquiisostearate (HLB = 4.5), sorbitan tristearate (HLB = 2.1), sorbitan sesquioleate (HLB = 3.7), sorbitan monooleate (HLB = 4.7), sorbitan trioleate (HLB = 1.7) and sorbitan monolaurate (HLB = 4.7).

Among these sorbitan fatty acid esters, sorbitan fatty acid esters having an HLB of 1 to 6 are preferred, and sorbitan fatty acid esters having an HLB of 3 to 5.5 and having a branched fatty acid group having 10 to 20 carbon atoms are more preferred to provide stable oil-in-water emulsion systems with a mannosyl erythritol lipid. Preferred examples include sorbitan monoisostearate (HLB = 5), sorbitan sesquistearate (HLB = 4.2), sorbitan sesquiisostearate (HLB = 4.5), sorbitan sesquioleate (HLB = 3.7), sorbitan monooleate (HLB 4.7) and sorbitan monolaurate (HLB = 4.7).

The content of the component (B) in the oil-in-water emulsion composition of the present invention is preferably 0.01 to 20 mass%, more preferably 0.1 to 12 mass%, even more preferably 1 to 8 mass%, to provide a stable oil-in-water emulsion system and in terms of irritation.

The oil-in-water emulsion composition of the present invention is desirably substantially free of a surfactant having a polyalkylene oxide group in terms of safety and environmental burdens, because a stable oil-in-water emulsion system can be provided even if the composition does not comprise a surfactant having a polyalkylene oxide group. In addition, the composition may be substantially free of a surfactant having an HLB of 8 or more, because a stable oil-in-water emulsion system can be provided even if the composition does not comprise a surfactant having high hydrophilicity, in particular, having an HLB of 8 or more. Here, "substantially free" means that the composition may comprise, relative to the total amount of the composition, 1 mass% or less, preferably 0.5 mass% or less, more preferably 0.01 mass% or less of the relevant component.

The mannosyl erythritol lipid (C) used in the present invention (hereinafter also called MEL) is a natural surfactant made by yeast, and is a compound having a structure in which erythritol is substituted at the 1-position of mannose, acyl groups are substituted at the 2- and 3-positions of mannose and acetyl groups are optionally substituted at the 4- and 6-positions of mannose.

Four known MELs based on the presence or absence of acetyl groups at the 4- and 6-positions of mannose are MEL-A, MEL-B, MEL-C and MEL-D. The formula (I) shows the structure of MEL-A. In the formula (I), $R^1$ and $R^2$ represent hydrocarbon groups. Specifically, MEL-A is a compound of the formula (I) having acyl groups having 1 to 19 carbon atoms at the 2- and 3-positions of mannose, and having acetyl groups at the 4- and 6-positions of mannose. MEL-B is a compound of the formula (I) where the acetyl group ($CH_3CO$) at the 4-position of mannose is replaced with H. MEL-C is a compound of the formula (I) where the acetyl group ($CH_3CO$) at the 6-position of mannose is replaced with H. MEL-D is a compound of the formula (I) where the acetyl groups ($CH_3CO$) at the 4- and 6-positions of mannose are both replaced with H.

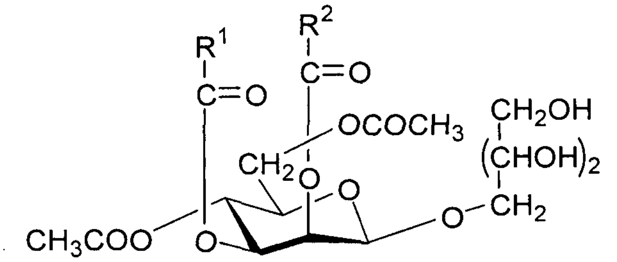

(I)

MEL-A

In formula (I), $R^1$ and $R^2$ are the same or different and each represents a hydrogen atom, a linear or branched alkyl group having 1 to 18 carbon atoms, or a linear or branched alkenyl, alkadienyl or alkatrienyl group having 2 to 18 carbon atoms.

In accordance with the formula (I), MEL is a compound having saturated or unsaturated, linear or branched acyl groups having 1 to 19 carbon atoms at the 2- and 3-positions of mannose, and having acetoxy groups at the 4- and 6-positions of mannose (either one or both of acetoxy groups at the 4- and 6-positions of mannose may be a hydroxy group(s)). The total number of carbon atoms in the acyl groups at the 2- and 3-positions is preferably 10 to 38, more preferably 14 to 30.

Preferred groups for $R^1$ and $R^2$ include $C_7$-$C_{11}$ alkyl groups such as $CH_3(CH_2)_6$, $CH_3(CH_2)_8$ and $CH_3(CH_2)_{10}$; $C_7$-$C_{11}$ alkenyl groups such as $C_7H_{14}$, $C_9H_{18}$ and $C_{11}H_{22}$; $C_7$-$C_{11}$ alkadienyl groups such as $C_7H_{12}$, $C_9H_{16}$ and $C_{11}H_{20}$; and $C_7$-$C_{11}$ alkatrienyl groups such as $C_7H_{10}$, $C_9H_{14}$ and $C_{11}H_{18}$.

Commercially available products such as SurfMellow BBG (manufactured by Toyobo Co., Ltd.; comprising 50 mass% of MEL-B: HLB = 8.7) can be used as such MELs.

The content of the component (C) in the oil-in-water emulsion composition of the present invention is preferably 0.005 to 8 mass%, more preferably 0.01 to 5 mass%, even more preferably 0.05 to 1 mass%. Mannosyl erythritol lipids are expensive and also may generate odor issuing from raw materials, because they are produced by fermenting vegetable oils, vegetable proteins or the like. If the content is within this range, a stable oil-in-water emulsion system is provided, usability is high, and odor issuing from raw materials is slight, and therefore the content within this range is preperable.

The surfactants in the oil-in-water emulsion composition of the present invention have a combined HLB of less than 8. By setting the combined HLB to be less than 8, a stable oil-in-water emulsion composition can be provided, although the composition is blended with a sufficient amount of an UV absorber. As described above, it is quite unexpected that stable oil-in-water emulsion compositions can be provided in spite of the low combined HLB of the surfactants of less than 8.

In the case of a high combined HLB, the composition becomes a water-in-oil emulsion composition rather than an oil-in-water emulsion composition, resulting in a strong oily feel.

Such combined HLB of the surfactants is preferably less than 8, more preferably 4 to 7.5, even more preferably 4.5 to 7.

The HLB of a surfactant mixture composed of two or more surfactants is determined as follows. The HLB of the surfactant mixture is obtained by arithmetic mean of the HLB values of the respective surfactants based on the blending ratio.

$$\text{Combined HLB} = \Sigma(\text{HLBx} \times \text{Wx}) \;/\; \Sigma\text{Wx}$$

HLBx represents the HLB value of a surfactant X.

Wx represents the mass (g) of the surfactant X having the HLBx value.

Example of components other than the above components in the oil-in-water emulsion composition of the present invention include, but are not limited to, water; color pigments such as tar pigments and iron oxide; preservatives such as paraben and phenoxyethanol; anionic surfactants such as sodium cetyl sulfate and N-stearoyl-L-glutamate; nonionic surfactants such as polyhydric alcohol fatty acid ester, polyglycerol fatty acid ester, modified silicone and sucrose ester; cationic surfactants such as tetraalkylammonium salt; amphoteric surfactants such as betaine, sulfobetaine and sulfoamino acid surfactants; natural surfactants such as lecithin, lysophosphatidylcholine, ceramide and cerebroside; pigments such as titanium oxide and zinc oxide; antioxidants such as dibutylhydroxytoluene; inorganic salts such as sodium chloride, magnesium chloride, sodium sulfate and potassium nitrate; organic acid salts such as sodium citrate,

potassium acetate, sodium succinate, sodium aspartate, sodium lactate, carnitine salt, γ-aminobutyric acid and lipoic acid; salts such as ethanolamine hydrochloride, ammonium nitrate and arginine hydrochloride; chelators such as edetic acid; neutralizers such as potassium hydroxide, diisopropanolamine and triethanolamine; biopolymers such as hyaluronic acid and collagen; placenta extract; UV absorbers such as hydroxymethoxybenzophenone sulfonate; vitamin A and derivatives thereof such as retinol, retinol acetate and retinol palmitate; vitamin E and derivatives thereof such as α-tocopherol, γ-tocopherol, δ-tocopherol, tocopherol nicotinate and tocopherol acetate; oil-soluble vitamin C derivatives such as ascorbyl palmitate, ascorbyl stearate and ascorbyl tetraisostearate; water-soluble polymers such as polysaccharides extracted from xanthan gum, β-glucan, oat, white jelly fungus and the like, polymers extracted from seaweed, like carrageenan, alginic acid and agar, carboxyvinyl polymers, pectin, and alkyl-modified carboxyvinyl polymers; and polyhydric alcohols such as dipropylene glycol, 1,3-butylene glycol, glycerol, propylene glycol, sorbitol, maltitol, diglycerol, raffinose and hexylene glycol.

Plant, seaweed or bacterial extracts can be used for the oil-in-water emulsion compositions of the present invention, and are, for example, extracts extracted from entire plant, leaves, stems, roots, fruits, seeds, flowers, fruit bodies and bacterial cells of artichoke, indigo plant, arnica, aloe, althea, Angelica keiskei, acerola, apricot, almond, Hydrangea macrophylla var. thunbergii, chocolate vine, anise, avocado, Artemisia capillaris flower, nettle, strawberry, fennel, turmeric, opuntia, oolong tea, common mallow, rosa multiflora, Echinacea angustifolia, oat, Isodon japonicus, edelweiss, watercress, Phellodendron amurense, Scutellaria baicalensis, Coptis japonica, giant crape myrtle, Hypericum erectum, orange, okra, olive leaf, black currant, Japanese valerian, persimmon, Pyracantha fortuneana, camomile, camu camu, carotte, Artemisia capillaris, Avena fatua, licorice, cucumber, apricot kernel, kiwi, cinchona, quillai, Hedera rhombea, gaba tea, raspberry, shrubby sophora, Sasa veitchii, mulberry, walnut, grapefruit, Geranium thunbergii, gentian, shell ginger, kothalahimbutu, burdock, comfrey, wheat germ, cherry blossom, common soapwort, salvia, Japanese hawthorn, Asarum sieboldii, umbilical cord, Gardenia fruit, meadowsweet, Houttuynia cordata, Spanish flag, sweet flag, ginger, Lithospermum purpurocaeruleum, perilla, white birch, peony, Rehmannia glutinosa, Citrus depressa, simon batatas, field horsetail, star fruit, high mallow, cnidii rhizoma, hawthorn, English ivy, pear, white willow, sage, Swertia japonica, soybean, bitter orange, thyme, Citrus tachibana, garden thyme, tamarind, tea, clove, citrus unshiu peel, camellia, Houttuynia cordata, Angelica acutiloba, peach kernel, bitter orange peel, tomato, garden marigold, Ampelopsis grossedentata, Potentilla erecta, corn, neem, horehound, elder, garlic, carrot, wild rose, Myristica argentea, hibiscus, parsley, banana, rose, Job's tears, lupine, pecan nuts, Japanese cypress, hyssop, Himalayan raspberry, herb Robert, sandalwood, bilberry, loquat, prune, grape, bunchflower daffodil, butterfly bush, rose apple, peppermint, safflower, sponge gourd, ribwort plantain, white genepi, Magnolia obovata, linden, Peucedanum japonicum, peony, hop, jojoba, quince, Rosa rugosa, horse-chestnut, pine, orange, soapberry, melissa, common evening primrose, peach, maple, cornflower, saxifrage, eucalyptus, lily, yuzu, coix seed, mugwort, orchid, lime, lavender lettuce, apple, Artemisia campestris, rooibos, milk vetch, lemon, lemon balm, rose hip, rosemary, green algae, red algae, brown algae, Poria sclerotium, shiitake mushroom, Hypholoma sublateritium, polypore, snow fungus, Ganoderma lucidum, plant worm, yeast, lactic acid bacteria and root nodule bacteria with water, organic solvents such as glycerol, propylene glycol, ethanol and butylene glycol, or mixtures thereof, or oils such as olive oil and macadamia nut oil. Chlorophylls are also plant extracts. Also, culture solutions of lactic acid bacteria or yeast using raw milk, fruit juice, synthetic media or semisynthetic media may be used regardless of removal of the bacterial cells.

The oil-in-water emulsion compositions of the present invention are superior in a feel upon application to the skin, for example, spreadability, and excellent in a feel such as freshness. Accordingly, the oil-in-water emulsion compositions of the present invention can be used for applications such as cosmetics and pharmaceuticals, and can be suitably applied as skin external preparations for sun protection such as sunscreen (such as emulsion, cream and essence), makeup base, foundation and lip cream, for example.

Specific examples of preferred embodiments of the present invention are shown below.

[1] An oil-in-water emulsion composition, comprising the following components (A) to (C):

(A) an UV absorber of the following (i) and/or (ii):

(i) at least one methoxycinnamic acid derivative selected from the group consisting of ethyl p-methoxycinnamate, isopropyl p-methoxycinnamate, 2-ethoxyethyl p-methoxycinnamate, 2-ethylhexyl p-methoxycinnamate, sodium p-methoxycinnamate, potassium p-methoxycinnamate and glyceryl mono-2-ethylhexanoate di-p-methoxycinnamate,
(ii) at least one benzoic acid derivative selected from the group consisting of p-aminobenzoic acid, ethyl p-aminobenzoate, glyceryl p-aminobenzoate, octyl p-aminobenzoate, amyl p-dimethylaminobenzoate, 2-ethylhexyl p-dimethylaminobenzoate and ethyl 4-[N,N-di(2-hydroxypropyl)amino]benzoate,

(B) a sorbitan fatty acid ester having an HLB of 6 or less, and

(C) a mannosyl erythritol lipid,

wherein the surfactants in the composition have a combined HLB of less than 8, and the content of the component (A) is 75 parts by mass or more relative to 100 parts by mass of the total amount of the oily components.
[2] The oil-in-water emulsion composition according to [1], wherein the component (A) is 2-ethylhexyl p-methoxycinnamate and/or 2-ethylhexyl p-dimethylaminobenzoate.
[3] The oil-in-water emulsion composition according to [1] or [2], wherein the sorbitan fatty acid ester of the component (B) is at least one selected from the group consisting of sorbitan monostearate, sorbitan monoisostearate, sorbitan sesquiisostearate, sorbitan sesquioleate, sorbitan monooleate, sorbitan trioleate and sorbitan monolaurate.
[4] The oil-in-water emulsion composition according to any of [1] to [3], wherein the component (B) is a sorbitan fatty acid ester having an HLB of 1 to 6.
[5] The oil-in-water emulsion composition according to any of [1] to [4], wherein the surfactants in the composition have a combined HLB of 4 to 7.5.
[6] The oil-in-water emulsion composition according to any of [1] to [5], wherein the surfactants in the composition have a combined HLB of 4.5 to 7.
[7] The oil-in-water emulsion composition according to any of [1] to [6], wherein the component (B) is a sorbitan fatty acid ester having an HLB of 3 to 5.5 and having a branched fatty acid group having 10 to 20 carbon atoms.
[8] The oil-in-water emulsion composition according to any of [1] to [7], wherein the component (A) is 2-ethylhexyl p-methoxycinnamate.
[9] The oil-in-water emulsion composition according to any of [1] to [8], which is substantially free of a surfactant having a polyalkylene oxide group.
[10] The oil-in-water emulsion composition according to any of [1] to [9], which is substantially free of a surfactant having an HLB of 8 or more.
[11] The oil-in-water emulsion composition according to any of [1] to [10], wherein the content of the component (A) is 1 to 40 mass%.
[12] The oil-in-water emulsion composition according to any of [1] to [11], wherein the content of the component (A) is 2 to 35 mass%.
[13] The oil-in-water emulsion composition according to any of [1] to [12], wherein the content of the component (A) is 3 to 25 mass%.
[14] The oil-in-water emulsion composition according to any of [1] to [13], wherein the content of the oily components (including the component (A)) is 1 to 40 mass%.
[15] The oil-in-water emulsion composition according to any of [1] to [14], wherein the content of the oily components (including the component (A)) is 2 to 35 mass%.
[16] The oil-in-water emulsion composition according to any of [1] to [15], wherein the content of the oily components (including the component (A)) is 3 to 30 mass%.
[17] The oil-in-water emulsion composition according to any of [1] to [16], wherein the content of the component (B) is 0.01 to 20 mass%.
[18] The oil-in-water emulsion composition according to any of [1] to [17], wherein the content of the component (B) is 0.1 to 12 mass%.
[19] The oil-in-water emulsion composition according to any of [1] to [18], wherein the content of the component (C) is 0.005 to 8 mass%.
[20] The oil-in-water emulsion composition according to any of [1] to [19], wherein the content of the component (C) is 0.01 to 5 mass%.
[21] The oil-in-water emulsion composition according to any of [1] to [20], wherein the content of the component (C) is 0.02 to 1 mass%.
[22] The oil-in-water emulsion composition according to any of [1] to [21], wherein the oil-in-water emulsion composition is a cosmetic composition for skin.

[Examples]

The present invention will be described in detail below based on examples and comparative examples; however, the present invention is not limited thereto. First, the sensory test used in examples and comparative examples will be described below.

[Sensory test]

Samples were used by 20 adult women evaluation panelists, and the evaluation was performed after use based on the number of panelists who answered that the samples gave an excellent feel, in terms of smooth feel upon application

(spreadability) and no oily feel after application (freshness). For Examples 1 to 10 and Comparative Examples 1 to 10, the presence or absence of ordor issuing from raw materials was evaluated based on the number of panelists who answered that they felt no particular ordor issuing from raw materials.

Examples 1 to 11, Comparative Examples 1 to 16

Sunscreens were prepared with the compositions described in Tables 1 and 2, and the above sensory test was performed.

(1) Preparation Method

The respective components are homogeneously mixed and stirred at room temperature.

(2) Properties

The results of the sensory test immediately after the manufacture in examples and comparative examples are shown in Tables 1 and 2.

[Table 1]

| Component | Example | | | | | | | | | | Comparative Example | | | | | | | | | |
|---|---|---|---|---|---|---|---|---|---|---|---|---|---|---|---|---|---|---|---|---|
| | 1 | 2 | 3 | 4 | 5 | 6 | 7 | 8 | 9 | 10 | 1 | 2 | 3 | 4 | 5 | 6 | 7 | 8 | 9 | 10 |
| 2-Ethylhexyl p-methoxycinnamate | 20 | 20 | 20 | 20 | | 20 | 20 | 4 | 10 | 30 | 20 | 20 | | | 20 | 20 | 20 | 20 | 20 | 20 |
| 2-Ethylhexyl p-dimethylaminobenzoate | | | | | 20 | | | | | | | | | | | | | | | |
| Methylphenylpolysiloxane | | | | | | | | | | | | | 20 | 20 | | | | | | |
| Sorbitan isostearate [HLB=5] | 4 | | | | 4 | 4 | 4 | 4 | 4 | 4 | | | 4 | 4 | 5 | 4 | | | 2 | |
| Sorbitan oleate [HLB=4.3] | | 4 | | | | | | | | | | | | | | | | | | 3 |
| Sorbitan laurate [HLB=4.7] | | | 4 | | | | | | | | | | | | | | | | | |
| Sorbitan stearate [HLB=4.7] | | | | 4 | | | | | | | | | | | | | | | | |
| Mannosyl erythritol lipid *1 | 0.8 | 0.8 | 0.8 | 0.8 | 0.8 | 0.2 | 8 | 0.8 | 0.8 | 0.8 | 0.8 | 8 | 0.8 | 8 | | | 0.8 | 0.8 | 0.8 | 0.8 |
| POE hydrogenated castor oil (60 E.O.) [HLB=14] | | | | | | | | | | | | | | | | 0.8 | 2 | | 2 | 2 |
| Cetyl dimethicone copolyol [HLB=5] *2 | | | | | | | | | | | | | | | | | | 5 | | |
| Pure water | 75.2 | 75.2 | 75.2 | 75.2 | 75.2 | 75.8 | 68 | 91.2 | 85.2 | 65.2 | 79.2 | 72 | 75.2 | 68 | 75 | 75.2 | 77.2 | 74.2 | 75.2 | 74.2 |
| Combined HLB | 5.34 | 4.7 | 5.06 | 5.06 | 5.34 | 5.09 | 6.85 | 5.34 | 5.34 | 5.34 | 8.7 | 8.7 | 5.34 | 6.85 | 5 | 5 | 13.1 | 5.27 | 9.43 | 8.22 |
| Test results Emulsion type | O/W | O/W | O/W | O/W | O/W | O/W | O/W | O/W | O/W | O/W | Separation | Separation | W/O | W/O | W/O | W/O | O/W | W/O | O/W | O/W |
| Smooth feel upon application | 19 | 18 | 18 | 18 | 18 | 17 | 17 | 20 | 20 | 18 | 2 | 1 | 1 | 0 | 2 | 1 | 18 | 0 | 14 | 14 |
| No Oily feel | 18 | 18 | 19 | 18 | 18 | 17 | 17 | 20 | 19 | 17 | 3 | 3 | 4 | 3 | 3 | 3 | 4 | 2 | 6 | 7 |
| Absence of odor issuing from raw materials | 19 | 18 | 16 | 18 | 19 | 20 | 16 | 20 | 18 | 17 | 17 | 15 | 18 | 14 | 17 | 17 | 18 | 15 | 18 | 17 |

*1 SurfMellow B BG manufactured by Toyobo Co., Ltd. (50% 1,3-butylene glycol solution) [HLB=8.7]

*2 ABIL EM90 manufactured by Evonik Industries

[Table 2]

| Component | Example | | Comparative Example | | | | | |
|---|---|---|---|---|---|---|---|---|
| | 1 | 11 | 11 | 12 | 13 | 14 | 15 | 16 |
| 2-Ethylhexyl p-methoxycinnamate | 20 | 16 | | | | | 14 | 11 |
| Methylphenylpolysiloxane | | 4 | | | | | 6 | 9 |
| Dimethylpolysiloxane | | | 20 | | | | | |
| Squalane | | | | 20 | | | | |
| Liquid paraffin | | | | | 20 | | | |
| α-Olefin oligomer | | | | | | 20 | | |
| Sorbitan isostearate [HLB=5] | 4 | 4 | 4 | 4 | 4 | 4 | 4 | 4 |
| Mannosyl erythritol lipid *1 | 0.8 | 0.8 | 0.8 | 0.8 | 0.8 | 0.8 | 0.8 | 0.8 |
| Pure water | 75.2 | 75.2 | 75.2 | 75.2 | 75.2 | 75.2 | 75.2 | 75.2 |
| | | | | | | | | |
| Test results Emulsion type | O/W | O/W | W/O | W/O | W/O | W/O | W/O | W/O |
| Smooth feel upon application | 19 | 18 | 3 | 2 | 1 | 1 | 1 | 3 |
| No Oily feel | 18 | 17 | 2 | 0 | 0 | 1 | 1 | 1 |
| *1 SurfMellow B BG manufactured by Toyobo Co., Ltd. (50% 1,3-butylene glycol solution) [HLB=8.7] | | | | | | | | |

The results of the sensory test immediately after the manufacture in examples and comparative examples are shown in Tables 1 and 2. The appearance of the samples was observed by microscopy to determine the emulsion type. As shown in Tables 1 and 2, in the examples according to the present invention, oil-in-water sunscreen compositions were formed and were excellent in a feel upon application such as spreadability and no oily feel. On the other hand, in the comparative examples, oil-in-water emulsion compositions were not formed, and feel of the resulting compositions was not satisfactory. In the Comparative Examples 1 and 2, separation occurred in the formulations and therefore the sensory test could not be performed.

Example 12 (sunscreen milk)

A sunscreen milk was prepared with the composition described in Table 3, and the above sensory test was performed.

[Table 3]

| Skin milk | |
|---|---|
| Component | Content (%) |
| Sorbitan isostearate | 2 |
| Mannosyl erythritol lipid *1 | 0.3 |
| 2-Ethylhexyl p-methoxycinnamate | 6 |
| Macadamia nut oil | 1 |
| Olive oil *2 | 0.1 |
| Dimethylpolysiloxane (6 cs) | 0.1 |
| 4-tert-Butyl-4'-methoxybenzoylmethane *3 | 0.1 |
| Argan oil | 0.1 |
| Octyldodecyl myristate | 0.05 |
| Dipropylene glycol | 2 |

(continued)

| Skin milk | |
|---|---|
| Component | Content (%) |
| Decaglyceryl diisostearate | 0.05 |
| Raffinose | 0.5 |
| Glycerol | 5 |
| Sorbitol solution (comprising 70% sorbitol) | 3 |
| Sorbic acid | 0.3 |
| $\gamma$-Amino-$\beta$-hydroxybutyric acid | 0.2 |
| N-Methyl-L-serine | 0.5 |
| Euglena gracilis polysaccharide | 0.2 |
| Hibiscus extract | 0.2 |
| Oil-soluble licorice extract | 0.01 |
| Avocado extract | 0.2 |
| Oolong tea extract | 0.1 |
| Phellodendron amurense extract | 1 |
| Polyethylene glycol 4000 | 1 |
| Carboxyvinyl polymer | 0.2 |
| Cherry blossom extract | 0.05 |
| Apricot kernel extract | 0.1 |
| Pear juice fermented liquor | 0.2 |
| Magnesium ascorbate phosphate | 0.01 |
| Chlorphenesin | 0.05 |
| Edetate | 0.02 |
| Potassium hydroxide | 0.05 |
| Phenoxyethanol | 0.2 |
| Flavor | 0.02 |
| Purified water | Balance |
| Test results Smooth feel upon application | 18 |
| No Oily feel | 18 |
| *1 SurfMellow B BG manufactured by Toyobo Co., Ltd. (50% 1,3-butylene glycol solution)<br>*2 Cropure OL manufactured by Croda Japan<br>*3 Parsol 1789 manufactured by Roche | |

(1) Preparation Method

The respective components are homogeneously mixed and stirred at room temperature.

(2) Properties

The above properties were tested for the composition of the test example. The results are shown in Table 3. As shown in Table 3, the composition of the example according to the present invention was superior in a feel upon application.

Example 13 (sunscreen cream)

A sunscreen cream was prepared with the composition described in Table 4, and the above sensory test was performed.

[Table 4]

| Skin cream | |
|---|---|
| Component | Content (%) |
| Sorbitan isostearate | 4 |
| Mannosyl erythritol lipid *1 | 1 |
| 2-Ethylhexyl p-methoxycinnamate | 7 |
| Avocado oil | 0.5 |
| Rice squalane | 0.1 |
| Dimethylpolysiloxane (20 cs) | 0.1 |
| Macadamia nut fatty acid phytosteryl ester | 0.01 |
| Long-chain branched fatty acid cholesteryl ester | 0.01 |
| Methylphenylpolysiloxane | 0.05 |
| Alkyl-modified carbomer *2 | 0.03 |
| Simulgel EG *3 | 1 |
| Propanediol | 2 |
| Ethanol | 5 |
| Sorbitol solution (comprising 70% sorbitol) | 3 |
| Polyethylene glycol 1000 | 1 |
| Polyoxyethylene hydrogenated castor oil (40 E.O.) | 0.1 |
| N-Methyl-L-serine | 0.5 |
| Carnitine chloride | 0.1 |
| Hibiscus fermented liquid | 0.2 |
| Bilberry leaf extract | 1 |
| Herb robert extract | 0.1 |
| Artichoke extract | 0.05 |
| Common evening primrose extract | 0.1 |
| Wheat germ extract | 0.1 |
| Rehmannia glutinosa extract | 0.1 |
| Peony extract | 0.1 |
| Rhododendrol | 0.2 |
| Magnesium ascorbate phosphate | 0.01 |
| p-Hydroxybenzoate | 0.3 |
| Chlorphenesin | 0.05 |
| Edetate | 0.02 |
| Potassium hydroxide | 0.1 |
| Phenoxyethanol | 0.2 |
| Flavor | 0.02 |

(continued)

| Skin cream | |
|---|---|
| Component | Content (%) |
| Purified water | Balance |
| Test results Smooth feel upon application | 17 |
| No Oily feel | 17 |
| *1 SurfMellow B BG manufactured by Toyobo Co., Ltd. (50% 1,3-butylene glycol solution)<br>*2 Pemulen TR-2 manufactured by B.F. Goodrich<br>*3 Manufactured by Seppic | |

(1) Preparation Method

The respective components are homogeneously mixed and stirred at room temperature.

(2) Properties

The above properties were tested for the composition of the test example. The results are shown in Table 4. As shown in Table 4, the composition of the example according to the present invention was superior in a feel upon use.

Example 14 (sunscreen moisturizing essence)

A sunscreen essence was prepared with the composition described in Table 5, and the above sensory test was performed.

[Table 5]

| Moisturizing essence | |
|---|---|
| Component | Content (%) |
| Sorbitan isostearate | 2 |
| Mannosyl erythritol lipid *1 | 0.1 |
| Octyl p-methoxycinnamate | 5 |
| Rice bran oil | 1 |
| Jojoba oil | 0.1 |
| Ethanol | 5 |
| Dimethylpolysiloxane (100 cs) | 0.1 |
| Isononyl isononanoate | 0.05 |
| 1,3-Butylene glycol | 2 |
| Carboxyvinyl polymer | 0.2 |
| Carboxymethylcellulose | 0.02 |
| Sodium polyacrylate | 0.1 |
| Xanthan gum | 0.05 |
| Carrageenan | 0.2 |
| Bentonite | 0.1 |
| Sodium ascorbate sulfate | 0.01 |
| Ascorbyl tetraisopalmitate | 0.1 |
| Retinol palmitate | 0.1 |

(continued)

| Moisturizing essence | |
|---|---|
| Component | Content (%) |
| Dipotassium glycyrrhizinate | 0.2 |
| Nicotinamide | 0.1 |
| γ-Aminobutyric acid | 0.1 |
| Diisopropylamine dichloroacetate | 0.2 |
| Camellia extract | 0.05 |
| Maple leaf extract | 0.1 |
| Saxifrage extract | 0.1 |
| Houttuynia cordata extract | 1 |
| Rose fruit extract | 0.1 |
| Plant worm extract | 0.1 |
| Althea extract | 0.2 |
| Chlorphenesin | 0.05 |
| Edetate | 0.02 |
| Potassium hydroxide | 0.05 |
| Phenoxyethanol | 0.2 |
| Flavor | 0.02 |
| Purified water | Balance |
| | |
| Test results Smooth feel upon application | 19 |
| No Oily feel | |
| *1 SurfMellow B BG manufactured by Toyobo Co., Ltd. (50% 1,3-butylene glycol solution) | |

(1) Preparation Method

The respective components are homogeneously mixed and stirred at room temperature.

(2) Properties

The above properties were tested for the composition of the test example. The results are shown in Table 5. As shown in Table 5, the composition of the example according to the present invention was superior in a feel upon application.

## Claims

**1.** An oil-in-water emulsion composition, comprising the following components (A) to (C):

(A) an UV absorber of the following (i) and/or (ii):

(i) at least one methoxycinnamic acid derivative selected from the group consisting of ethyl p-methoxycinnamate, isopropyl p-methoxycinnamate, 2-ethoxyethyl p-methoxycinnamate, 2-ethylhexyl p-methoxycinnamate, sodium p-methoxycinnamate, potassium p-methoxycinnamate and glyceryl mono-2-ethylhexanoate di-p-methoxycinnamate,
(ii) at least one benzoic acid derivative selected from the group consisting of p-aminobenzoic acid, ethyl p-aminobenzoate, glyceryl p-aminobenzoate, octyl p-aminobenzoate, amyl p-dimethylaminobenzoate, 2-ethylhexyl p-dimethylaminobenzoate and ethyl 4-[N,N-di(2-hydroxypropyl)amino]benzoate,

(B) a sorbitan fatty acid ester having an HLB of 6 or less, and
(C) a mannosyl erythritol lipid,

wherein the surfactants in the composition have a combined HLB of less than 8, and the content of the component (A) is 75 parts by mass or more relative to 100 parts by mass of the total amount of the oily components.

2. The oil-in-water emulsion composition according to claim 1, wherein the component (A) comprises at least one selected from the group consisting of 2-ethylhexyl p-methoxycinnamate, glyceryl mono-2-ethylhexanoate di-p-methoxycinnamate and 2-ethylhexyl p-dimethylaminobenzoate.

3. The oil-in-water emulsion composition according to claim 1 or claim 2, wherein the component (A) is 2-ethylhexyl p-methoxycinnamate and/or 2-ethylhexyl p-dimethylaminobenzoate.

4. The oil-in-water emulsion composition according to any of claims 1 to 3, wherein the component (C) is a compound represented by formula (I):

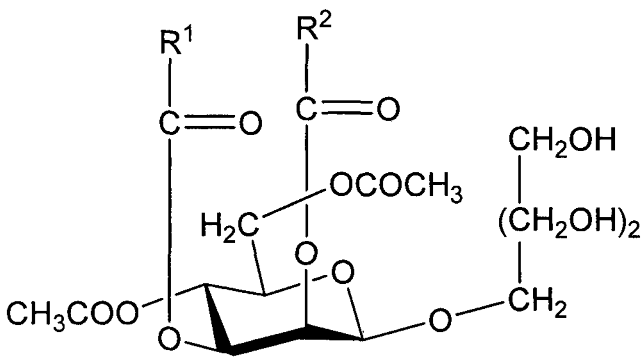

(I)

wherein $R^1$ and $R^2$ are the same or different and each represents a hydrogen atom, a linear or branched alkyl group having 1 to 18 carbon atoms, or a linear or branched alkenyl, alkadienyl or alkatrienyl group having 2 to 18 carbon atoms.

5. The oil-in-water emulsion composition according to any of claims 1 to 3, wherein the component (C) is a compound represented by formula (I'):

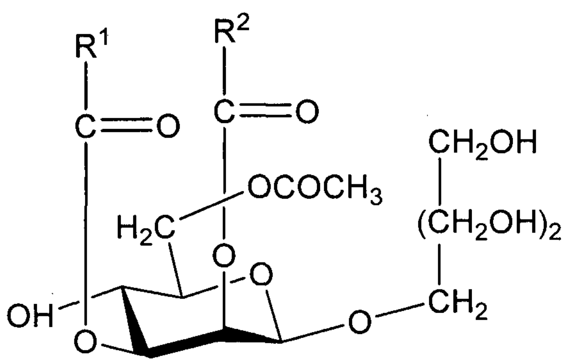

(I')

wherein $R^1$ and $R^2$ are the same or different and each represents a hydrogen atom, a linear or branched alkyl group having 1 to 18 carbon atoms, or a linear or branched alkenyl, alkadienyl or alkatrienyl group having 2 to 18 carbon atoms.

6. The oil-in-water emulsion composition according to any of claims 1 to 3, wherein the component (C) is a compound represented by formula (I"):

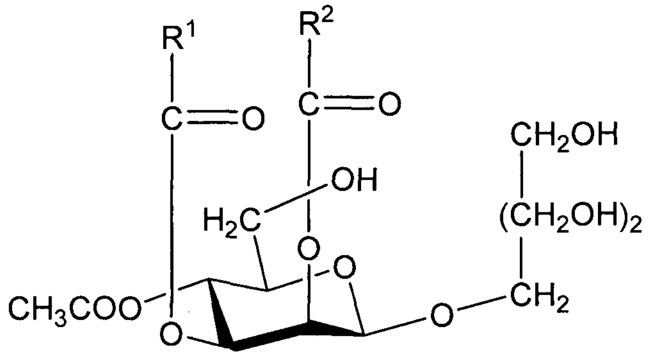

(I'')

wherein $R^1$ and $R^2$ are the same or different and each represents a hydrogen atom, a linear or branched alkyl group having 1 to 18 carbon atoms, or a linear or branched alkenyl, alkadienyl or alkatrienyl group having 2 to 18 carbon atoms.

7. The oil-in-water emulsion composition according to any of claims 1 to 3, wherein the component (C) is a compound represented by formula (I''') :

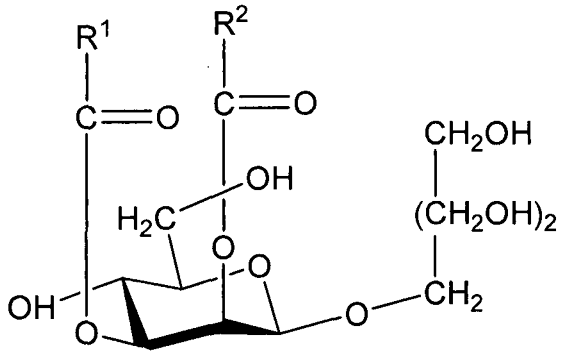

(I''')

wherein $R^1$ and $R^2$ are the same or different and each represents a hydrogen atom, a linear or branched alkyl group having 1 to 18 carbon atoms, or a linear or branched alkenyl, alkadienyl or alkatrienyl group having 2 to 18 carbon atoms.

8. The oil-in-water emulsion composition according to any of claims 4 to 7, wherein the total number of carbon atoms in the acyl groups at the 2- and 3-positions is 10 to 38.

9. The oil-in-water emulsion composition according to any of claims 4 to 7, wherein the total number of carbon atoms in the acyl groups at the 2- and 3-positions is 14 to 30.

10. The oil-in-water emulsion composition according to any of claims 1 to 9, wherein the component (B) is a sorbitan fatty acid ester having an HLB of 1 to 6.

11. The oil-in-water emulsion composition according to any of claims 1 to 10, wherein the content of the component (A) in the oil-in-water emulsion composition is 1 to 40 mass%.

12. The oil-in-water emulsion composition according to any of claims 1 to 11, wherein the content of the component (B) in the oil-in-water emulsion composition is 0.01 to 20 mass%.

13. The oil-in-water emulsion composition according to any of claims 1 to 12, wherein the content of the component (C) in the oil-in-water emulsion composition is 0.005 to 8 mass%.

14. The oil-in-water emulsion composition according to any of claims 1 to 13, wherein the surfactants in the oil-in-water emulsion composition have a combined HLB of 4 to 7.5.

15. The oil-in-water emulsion composition according to any of claims 1 to 14, wherein the content of the component (A) in the oil-in-water emulsion composition is 80 to 100 parts by mass, relative to 100 parts by mass of the total amount of the oily components.

16. The oil-in-water emulsion composition according to any of claims 1 to 15, wherein the content of a surfactant having a polyalkylene oxide group in the oil-in-water emulsion composition is 1 mass% or less.

**Patentansprüche**

1. Öl-in-Wasser-Emulsionszusammensetzung, enthaltend die folgenden Komponenten (A) bis (C):

(A) einen UV-Absorber gemäß (i) und/oder (ii) :

(i) zumindest ein Methoxyzimtsäurederivat, ausgewählt aus der Gruppe bestehend aus Ethyl-p-methoxycinnamat, Isopropyl-p-methoxycinnamat, 2-Ethoxyethyl-p-methoxycinnamat, 2-Ethylhexyl-p-methoxycinnamat, Natrium-p-methoxycinnamat, Kalium-p-methoxycinnamat und Glyceryl-mono-2-ethylhexanoat-di-p-methoxycinnamat,
(ii) zumindest ein Benzoesäurederivat, ausgewählt aus der Gruppe bestehend aus p-Aminobenzoesäure, Ethylp-aminobenzoat, Glyceryl-p-aminobenzoat, Octyl-p-aminobenzoat, Amyl-p-dimethylaminobenzoat, 2-Ethylhexylp-dimethylaminobenzoat und Ethyl-4-[N,N-di(2-hydroxypropyl)amino]benzoat,

(B) einen Sorbitanfettsäureester mit einem HLB von 6 oder weniger und
(C) ein Mannosylerythritlipid,

worin die Tenside in der Zusammensetzung einen kombinierten HLB von weniger als 8 haben und der Gehalt der Komponente (A) 75 Massenteile oder mehr in Bezug auf 100 Massenteile der Gesamtmenge der öligen Komponenten ist.

2. Öl-in-Wasser-Emulsionszusammensetzung nach Anspruch 1, worin die Komponente (A) zumindest eines enthält, ausgewählt aus der Gruppe bestehend aus 2-Ethylhexyl-p-methoxycinnamat, Glyceryl-mono-2-ethylhexanoat-di-p-methoxycinnamat und 2-Ethylhexyl-p-dimethylaminobenzoat.

3. Öl-in-Wasser-Emulsionszusammensetzung nach Anspruch 1 oder 2, worin die Komponente (A) 2-Ethylhexyl-p-methoxycinnamat und/oder 2-Ethylhexyl-p-dimethylaminobenzoat ist.

4. Öl-in-Wasser-Emulsionszusammensetzung nach einem der Ansprüche 1 bis 3, worin die Komponente (C) eine Verbindung mit der Formel (I) ist:

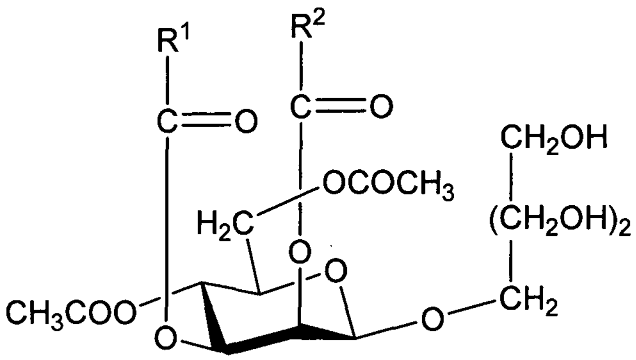

(I)

worin $R^1$ und $R^2$ gleich oder verschieden sind und jeweils ein Wasserstoffatom, eine lineare oder verzweigte Alkylgruppe mit 1 bis 18 Kohlenstoffatomen oder lineare oder verzweigte Alkenyl-, Alkadienyl- oder Alkatrienyl-Gruppe mit 2 bis 18 Kohlenstoffatomen sind.

5. Öl-in-Wasser-Emulsionszusammensetzung nach einem der Ansprüche 1 bis 3, worin die Komponente (C) eine Verbindung mit der Formel (I') ist:

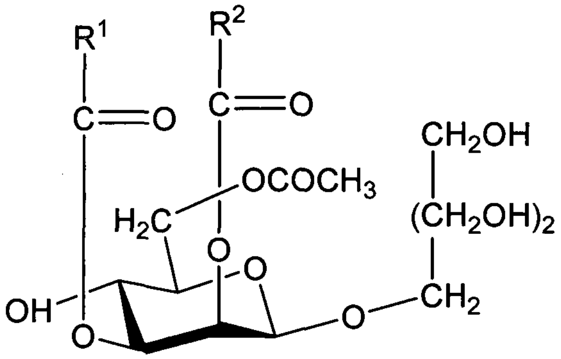

(I')

worin $R^1$ und $R^2$ gleich oder verschieden sind und jeweils ein Wasserstoffatom, eine lineare oder verzweigte Alkylgruppe mit 1 bis 18 Kohlenstoffatomen oder lineare oder verzweigte Alkenyl-, Alkadienyl- oder Alkatrienyl-Gruppe mit 2 bis 18 Kohlenstoffatomen sind.

**6.** Öl-in-Wasser-Emulsionszusammensetzung nach einem der Ansprüche 1 bis 3, worin die Komponente (C) eine Verbindung mit der Formel (I") ist:

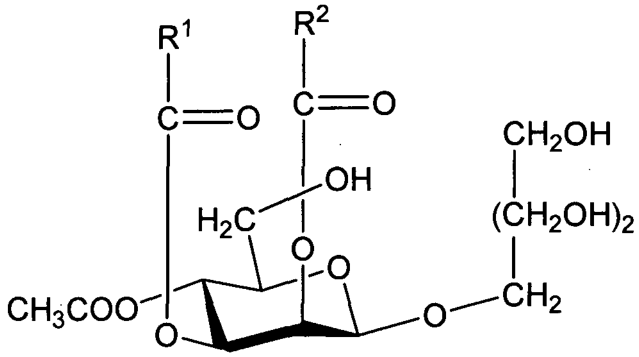

(I")

worin $R^1$ und $R^2$ gleich oder verschieden sind und jeweils ein Wasserstoffatom, eine lineare oder verzweigte Alkylgruppe mit 1 bis 18 Kohlenstoffatomen oder lineare oder verzweigte Alkenyl-, Alkadienyl- oder Alkatrienyl-Gruppe mit 2 bis 18 Kohlenstoffatomen sind.

**7.** Öl-in-Wasser-Emulsionszusammensetzung nach einem der Ansprüche 1 bis 3, worin die Komponente (C) eine Verbindung mit der Formel (I"') ist:

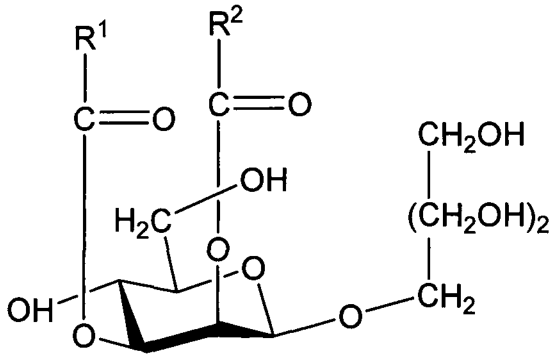

(I"')

worin $R^1$ und $R^2$ gleich oder verschieden sind und jeweils ein Wasserstoffatom, eine lineare oder verzweigte Alkylgruppe mit 1 bis 18 Kohlenstoffatomen oder lineare oder verzweigte Alkenyl-, Alkadienyl- oder Alkatrienyl-Gruppe mit 2 bis 18 Kohlenstoffatomen sind.

**8.** Öl-in-Wasser-Emulsionszusammensetzung nach einem der Ansprüche 4 bis 7, worin die Gesamtzahl der Kohlenstoffatome in der Acylgruppe an den 2- und 3-Positionen 10 bis 38 ist.

9. Öl-in-Wasser-Emulsionszusammensetzung nach einem der Ansprüche 4 bis 7, worin die Gesamtzahl der Kohlenstoffatome in der Acylgruppe an den 2- und 3-Positionen 14 bis 30 ist.

10. Öl-in-Wasser-Emulsionszusammensetzung nach einem der Ansprüche 1 bis 9, worin die Komponente (B) ein Sorbitanfettsäureester mit einem HLB von 1 bis 6 ist.

11. Öl-in-Wasser-Emulsionszusammensetzung nach einem der Ansprüche 1 bis 10, worin der Gehalt der Komponente (A) in der Öl-in-Wasser-Emulsionszusammensetzung 1 bis 40 Massen-% ist.

12. Öl-in-Wasser-Emulsionszusammensetzung nach einem der Ansprüche 1 bis 11, worin der Gehalt der Komponente (B) in der Öl-in-Wasser-Emulsionszusammensetzung 0,01 bis 20 Massen-% ist.

13. Öl-in-Wasser-Emulsionszusammensetzung nach einem der Ansprüche 1 bis 12, worin der Gehalt der Komponente (C) in der Öl-in-Wasser-Emulsionszusammensetzung 0,005 bis 8 Massen-% ist.

14. Öl-in-Wasser-Emulsionszusammensetzung nach einem der Ansprüche 1 bis 13, worin die Tenside in der Öl-in-Wasser-Emulsionszusammensetzung einen kombinierten HLB von 4 bis 7,5 haben.

15. Öl-in-Wasser-Emulsionszusammensetzung nach einem der Ansprüche 1 bis 14, worin der Gehalt der Komponente (A) in der Öl-in-Wasser-Emulsionszusammensetzung 80 bis 100 Massenteile in Bezug auf 100 Massenteile der Gesamtmenge der öligen Komponenten ist.

16. Öl-in-Wasser-Emulsionszusammensetzung nach einem der Ansprüche 1 bis 15, worin der Gehalt eines Tensides mit einer Polyalkylenoxidgruppe in der Öl-in-Wasser-Emulsionszusammensetzung 1 Massen-% oder weniger ist.

**Revendications**

1. Composition d'émulsion huile dans l'eau, comprenant les composants (A) à (C) suivants :

    (A) un absorbeur d'UV selon les (i) et/ou (ii) suivants :

        (i) au moins un dérivé de l'acide méthoxycinnamique choisi dans le groupe constitué de p-méthoxycinnamate d'éthyle, p-méthoxycinnamate d'isopropyle, p-méthoxycinnamate de 2-éthoxyéthyle, p-méthoxycinnamate de 2-éthylhexyle, p-méthoxycinnamate de sodium, p-méthoxycinnamate de potassium et mono-2-éthylhexanoate et di-p-méthoxycinnamate de glycéryle,
        (ii) au moins un dérivé de l'acide benzoïque choisi dans le groupe constitué d'acide p-aminobenzoïque, p-aminobenzoate d'éthyle, p-aminobenzoate de glycéryle, p-aminobenzoate d'octyle, p-diméthylaminobenzoate d'amyle, p-diméthylaminobenzoate de 2-éthylhexyle et 4-[N,N-di(2-hydroxypropyl)amino]benzoate d'éthyle,

    (B) un ester d'acide gras et de sorbitane ayant un EHL de 6 ou moins, et
    (C) un lipide de mannosylérythritol,

    dans laquelle les tensioactifs dans la composition ont un EHL combiné de moins de 8, et la teneur en composant (A) est de 75 parties en masse ou supérieure par rapport à 100 parties en masse de la quantité totale des composants huileux.

2. Composition d'émulsion huile dans l'eau selon la revendication 1, dans laquelle le composant (A) comprend au moins un choisi dans le groupe constitué de p-méthoxycinnamate de 2-éthylhexyle, mono-2-éthylhexanoate et di-p-méthoxycinnamate de glycéryle et p-diméthylaminobenzoate de 2-éthylhexyle.

3. Composition d'émulsion huile dans l'eau selon la revendication 1 ou la revendication 2, dans laquelle le composant (A) est le p-méthoxycinnamate de 2-éthylhexyle et/ou le p-diméthylaminobenzoate de 2-éthylhexyle.

4. Composition d'émulsion huile dans l'eau selon l'une quelconque des revendications 1 à 3, dans laquelle le composant

(C) est un composé représenté par la formule (I) :

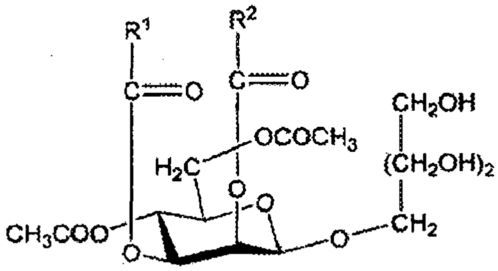

(I)

dans laquelle $R^1$ et $R^2$ sont identiques ou différents et chacun représente un atome d'hydrogène, un groupe alkyle linéaire ou ramifié ayant 1 à 18 atomes de carbone, ou un groupe linéaire ou ramifié alcényle, alcadiényle ou alcatriényle ayant 2 à 18 atomes de carbone.

**5.** Composition d'émulsion huile dans l'eau selon l'une quelconque des revendications 1 à 3, dans laquelle le composant (C) est un composé représenté par la formule (I') :

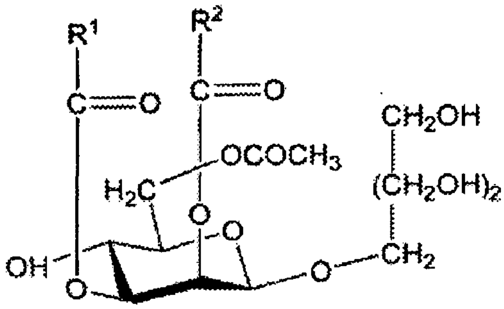

(I')

dans laquelle $R^1$ et $R^2$ sont identiques ou différents et chacun représente un atome d'hydrogène, un groupe alkyle linéaire ou ramifié ayant 1 à 18 atomes de carbone, ou un groupe linéaire ou ramifié alcényle, alcadiényle ou alcatriényle ayant 2 à 18 atomes de carbone.

**6.** Composition d'émulsion huile dans l'eau selon l'une quelconque des revendications 1 à 3, dans laquelle le composant (C) est un composé représenté par la formule (I") :

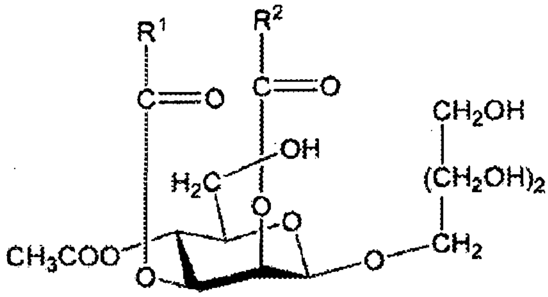

(I")

dans laquelle $R^1$ et $R^2$ sont identiques ou différents et chacun représente un atome d'hydrogène, un groupe alkyle linéaire ou ramifié ayant 1 à 18 atomes de carbone, ou un groupe linéaire ou ramifié alcényle, alcadiényle ou alcatriényle ayant 2 à 18 atomes de carbone.

**7.** Composition d'émulsion huile dans l'eau selon l'une quelconque des revendications 1 à 3, dans laquelle le composant (C) est un composé représenté par la formule (I"') :

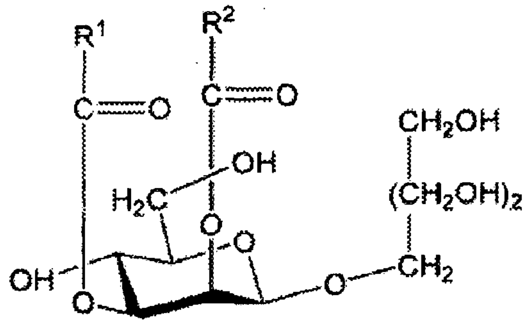

(I''')

dans laquelle $R^1$ et $R^2$ sont identiques ou différents et chacun représente un atome d'hydrogène, un groupe alkyle linéaire ou ramifié ayant 1 à 18 atomes de carbone, ou un groupe linéaire ou ramifié alcényle, alcadiényle ou alcatriényle ayant 2 à 18 atomes de carbone.

**8.** Composition d'émulsion huile dans l'eau selon l'une quelconque des revendications 4 à 7, dans laquelle le nombre total d'atomes de carbone dans les groupes acyle au niveau des positions 2 et 3 est de 10 à 38.

**9.** Composition d'émulsion huile dans l'eau selon l'une quelconque des revendications 4 à 7, dans laquelle le nombre total d'atomes de carbone dans les groupes acyle au niveau des positions 2 et 3 est de 14 à 30.

**10.** Composition d'émulsion huile dans l'eau selon l'une quelconque des revendications 1 à 9, dans laquelle le composant (B) est un ester d'acide gras et de sorbitane ayant un EHL de 1 à 6.

**11.** Composition d'émulsion huile dans l'eau selon l'une quelconque des revendications 1 à 10, dans laquelle la teneur en composant (A) dans la composition d'émulsion huile dans l'eau est de 1 à 40 % en masse.

**12.** Composition d'émulsion huile dans l'eau selon l'une quelconque des revendications 1 à 11, dans laquelle la teneur en composant (B) dans la composition d'émulsion huile dans l'eau est de 0,01 à 20 % en masse.

**13.** Composition d'émulsion huile dans l'eau selon l'une quelconque des revendications 1 à 12, dans laquelle la teneur en composant (C) dans la composition d'émulsion huile dans l'eau est de 0,005 à 8 % en masse.

**14.** Composition d'émulsion huile dans l'eau selon l'une quelconque des revendications 1 à 13, dans laquelle les tensioactifs dans la composition d'émulsion huile dans l'eau ont un EHL combiné de 4 à 7,5.

**15.** Composition d'émulsion huile dans l'eau selon l'une quelconque des revendications 1 à 14, dans laquelle la teneur en composant (A) dans la composition d'émulsion huile dans l'eau est de 80 à 100 parties en masse, par rapport à 100 parties en masse de la quantité totale des composants huileux.

**16.** Composition d'émulsion huile dans l'eau selon l'une quelconque des revendications 1 à 15, dans laquelle la teneur en tensioactif ayant un groupe poly(oxyde d'alkylène) dans la composition d'émulsion huile dans l'eau est de 1% en masse ou moins.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2009102236 A
- JP 2007153858 A
- JP 11262653 A
- JP 11071261 A
- JP 2009167159 A
- JP 2009149566 A
- JP 2009149567 A